# EUROPÄISCHE PATENTSCHRIFT

(11) **EP 2 754 614 B1**
(45) Veröffentlichungstag und Bekanntmachung des Hinweises auf die Patenterteilung: **24.06.2015**
(21) Anmeldenummer: 13151164.4
(22) Anmeldetag: 14.01.2013
(51) Int. Cl.: B65B 5/10, B65B 9/04, B65B 69/00, G06F 19/00

(54) **Anlage und Verfahren zur personenbezogenen Befüllung von Blisterpackungen mit Medikamenten**
System and method for personalised filling of blister packages with drugs
Installation et procédé destinés au remplissage pour une personne de blisters avec des médicaments

(43) Veröffentlichungstag der Anmeldung: 16.07.2014
(73) Patentinhaber: Kohl, Edwin, 66706 Perl (DE)
(72) Erfinder: Kohl, Edwin, 66706 Perl (DE)
(74) Vertreter: Betten & Resch

(56) Entgegenhaltungen:
- EP-A1- 2 062 822
- EP-A1- 2 343 243
- WO-A1-98/29084
- US-B1- 7 185 476

## Beschreibung

### Gebiet der Erfindung

Die Erfindung betrifft eine Anlage und ein Verfahren zur personenindividuellen Bestückung von Befüllblistern mit Medikamenten entsprechend vorgegebenen Verschreibungsdaten, wobei die Befüllblister in einer Matrixkonfiguration in Zeilen entsprechend einer Anzahl von Einnahmezeiten während eines Tages und Spalten entsprechend einer Anzahl von Tagen, beispielsweise Wochentagen, angeordnete Aufnahmefächer für jeweilige Medikamenten-Verabreichungseinheiten aufweisen.

### Verwandter Stand der Technik

Aus der DE 10 2004 034 024 A2 ist eine individuelle Blisterpackung für den wöchentlichen Medikamentenbedarf eines Patienten (Wochenblister) bekannt, der die Medikamente in einer nach Datum, Wochentag und Tageszeit (Morgen, Mittag und Abend und/oder Nacht) geordneten Folge einnehmen soll. Die Aufnahmefächer der Blisterpackung zur Aufnahme der jeweiligen Medikamente sind in Matrixform angeordnet mit sieben Tagesspalten mit jeweils mindestens drei Tageszeit-Fächern, die insgesamt durch eine zusammenhängende Blisterfolie verschlossen sind. An der Blisterpackung angebracht ist ein kartonförmiger Deckel, auf dem patientenindividuell die Zusammensetzung der einzelnen Tageszeit-Fächer sowie Verschreibungs-Informationen betreffend die jeweiligen Medikamente enthalten ist.

Derartige Wochenblister sind für Patienten, die regelmäßig mehrere verschiedene Medikamente einnehmen müssen, eine Hilfe dafür, einerseits das Einnehmen wichtiger Medikamente nicht zu vergessen und andererseits auch einen zuverlässigen Überblick darüber zu behalten, welche Medikamente schon eingenommen sind, um eine unter Umständen schädliche Übermedikation zu vermeiden.

Aus der WO 2005 102 841 A1 ist eine Anlage zur Bestückung von derartigen patientenindividuellen Blisterpackungen mit der gewünschten Medikamentenzusammenstellung bekannt. Für jedes Medikament ist eine Ausgabestation vorgesehen, die mittels den Aufnahmefächern der Blisterpackung räumlich zugeordneten Stösseln die jeweiligen Medikamente aus einer vorübergehenden Verpackung in Form eines Streifenblisters in die entsprechenden Aufnahmefächer der Verpackungseinheit befördert. Alle patientenindividuellen Blisterpackungen durchlaufen taktweise alle Ausgabestationen der Anlage, wobei nur an denjenigen Stationen Medikamente aufgenommen werden, die den der individuellen Blisterpackung zugeordneten Verschreibungsdaten entsprechen. So können patientenindividuelle Verpackungseinheiten mit einer Auswahl aus mehreren hundert Medikamenten vollautomatisch sicher und zuverlässig befüllt werden.

Da die von der Verpackungsanlage verarbeiteten Medikamente in sehr unterschiedliche Mengen verschrieben werden, können bei den seltener verschriebenen Medikamenten relativ lange "Standzeiten" in den vorübergehenden Verpackungen, d.h. den Streifenblistern auftreten. Damit die Medikamente nicht durch das Eindringen von Feuchtigkeit oder dgl. unbrauchbar werden, muß der Streifenblister daher eine gute Versiegelung des Aufnahmeraumes für das Medikament insbesondere gegenüber Wasserdampf aufweisen, Diese hat andererseits den Nachteil, dass sie bei Anwendung einer mechanischen Kraft auf die Medikamenteneinheit durch den Stössel nicht zuverlässig an der richtigen Stelle oder nicht vollständig aufreißt, so dass es zu Fehlern bei der Bestückung der Blisterpackung kommen kann. Da schon die falsche Befüllung eines Faches zwingend die Aussortierung der gesamten Blisterpackung bedingt, sind Fehler bei der "Umpackung" der Medikamente aus dem Streifenblister in die Blisterpackung mit hohem Aufwand und Kosten verbunden. Weiterhin ist nachteilig, dass die Wartung einer Medikamenten-Befüllstation aufgrund des Taktbetriebes zum Stillstand der ganzen Anlage führt.

Die EP 2 343 243 beschreibt Anlage zur überwachten Abfüllung fester pharmazeutischer Produkte in Blisternäpfe einer Blisterverpackung, mit einer Mehrzahl von Füllvorrichtungen zur Befüllung der Büsternäpfe mit festen pharmazeutischen Produkten;mindestens einem zwischen den Füllvorrichtungen bewegbaren Trans-portelement, das einen Lagerungsabschnitt zur Lagerung der Blisterverpackung aufweist, wobei das Transportelement im Bereich des Lagerungsabschnitts mehrere kapazitive Sensoren zur Bestimmung der Masse der in jedem Blisternapf enthaltenen pharmazeutischen Produkte aufweist.

### Zusammenfassung der Erfindung

Es ist daher eine Aufgabe der Erfindung, eine eine Anlage und ein Verfahren zur personenindividuellen Bestückung von Befüllblistern mit Medikamenten entsprechend vorgegebenen Verschreibungsdaten vorzuschlagen, die eine verbesserte Leistungsfähigkeit und Wartungsfreundlichkeit aufweist.

Gelöst wird die Aufgabe durch eine Anlage zur personenindividuellen Bestückung von Befüllblistern mit Medikamenten entsprechend vorgegebenen Verschreibungsdaten, wobei die Befüllblister in einer Matrixkonfiguration in Zeilen entsprechend einer Anzahl von Einnahmezeiten während eines Tages und Spalten entsprechend einer Anzahl von Tagen, beispielsweise Wochentagen, angeordnete Aufnahmefächer für jeweilige Medikamenten-Verabreichungseinheiten aufweisen, umfassend eine Vielzahl hintereinander angeordneter Medikamenten-Befüllstationen zur Bestückung der Befüllblister mit jeweils einem bestimmten Medikament, eine Transportvorrichtung ausgebildet zum Transport der Befüllblister einzeln hintereinander in einer Bewegungsrichtung entlang der Medikamenten-Befüllstationen, wobei jeder Befüllblister individuell gesteuert ist, derart dass dieser nur die entsprechend den Verschreibungsdaten jeweils erforderlichen Medikamenten-Befüllstationen für einen Befüllvorgang anfährt und die anderen Medikamenten-Befüllstationen passiert und direkt bis zur nächsten erforderlichen Medikamenten-Befüllstationen oder zu einer Position hinter einem an einer Medikamenten-Befüllstationen befindlichen vorausgehenden Befüllblister weiterbefördert wird dadurch **gekennzeichnet,** dass die Befüllblister (25) einzeln und hintereinander den gesamten Weg von der ersten bis zur letzten der Medikamenten-Befüllstationen (40) durchlaufen und die Geschwindigkeit der beförderten Befüllblister (25) einzeln ansteuerbar ist.

Die Aufgabe wird ferner gelöst duch ein Verfahren zur personenindividuellen Bestückung von Befüllblistern mit Medikamenten entsprechend vorgegebenen Verschreibungsdaten, wobei die Befüllblister in einer Matrixkonfiguration in Zeilen entsprechend einer Anzahl von Einnahmezeiten während eines Tages und Spalten entsprechend einer Anzahl von Tagen, beispielsweise Wochentagen, angeordnete Aufnahmefächer für jeweilige Medikamenten-Verabreichungseinheiten aufweisen, welches Verfahren die Förderung der Befüllblister einzeln hintereinander in einer Bewegungsrichtung entlang von hintereinander angeordneten Medikamenten-Befüllstationen zur Bestückung der Befüllblister mit jeweils einem bestimmten Medikament umfasst, wobei jeder Befüllblister individuell gesteuert wird, derart dass dieser nur die entsprechend den Verschreibungsdaten jeweils erforderlichen Medikamenten-Befüllstationen für einen Befüllvorgang anfährt und die anderen Medikamenten-Befüllstationen passiert und direkt bis zur nächsten erforderlichen Medikamenten-Befüllstationen oder zu einer Position hinter einem an einer Medikamenten-Befüllstationen befindlichen vorausgehenden Befüllblister weiterbefördert wird **dadurch gekennzeichnet,** dass die Befüllblister (25) einzeln und hintereinander den gesamten Weg von der ersten bis zur letzten der Medikamenten-Befüllstationen (40) durchlaufen und die Geschwindigkeit der beförderten Befüllblister (25) einzeln ansteuerbar ist.

Die individuelle Steuerung der einzelnen Befüllblister ohne festen Taktbetrieb nur an die entsprechend den Verschreibungsdaten jeweils erforderlichen Medikamenten-Befüllstationen ermöglicht einen schnelleren Durchlauf der einzelnen Befüllblister durch die Anlage und damit eine höhere Leistungsfähigkeit. Ausserdem wird der Betrieb der Anlage durch Wartungsarbeiten an einer einzelnen Medikamenten-Befüllstation nicht lahmgelegt.

Vorzugsweise werden die Befüllblister mittels einer Magnetbahn mit Linearmotor angetrieben, wodurch eine individuelle Ansteuerung und ein zuverlässiger Transport einer Vielzahl von Befüllblistern möglich ist. Vorzugsweise sind dabei in einer Magnetschiene geführte Werkstückträger zur Aufnahme der Befüllblister vorgesehen, welche Werkstückträger in einem geschlossenen Kreislauf gefördert werden, wobei die Befüllblister vor der Bestückung auf die Werkstückträger aufgesetzt und nach dem Bestückungsvorgang wieder entnommen werden.

Zur Steigerung der Leistungsfähigkeit der Anlage kann dabei in einem Arbeitsschritt jeweils eine Spalte eines Befüllblisters entsprechend einer Anzahl von Tagen gleichzeitig befüllt werden.

Vorzugsweise weist jede Befüllstation ein bewegbares Vordosiermagazin zur Befüllung der Befüllblister auf, wodurch der zeitkritische Vorgang der Befüllung des Befüllblisters von dem mechanisch schwierigen Vorgang des Ausstossens der Medikamenteinheiten aus dem Streifenblister entkoppelt wird.

Die Anlage kann ferner eine über der Transportvorrichtung angeordnete Kamera zur optischen Überprüfung der korrekten Bestückung der Befüllblister entsprechend den Verschreibungsdaten aufweisen. Mehrere Befüllstationen können dabei zu Befüllmodulen zusammengefasst sein, wobei nach jedem Befüllmodul eine solche Kamera vorgesehen ist.

Vorzugsweise ist ein Reinraumbereich zur Schaffung von Reinraumbedingungen bei der Bestückung der Befüllblister vorgesehen.

Das erfindungsgemäße Verfahren weist vorzugsweise den Verfahrensschritt des Erstellens und Anfügens eines Dokumentationsträgers an den Befüllblister umfassend personenbezogene Information und/oder Anwendungsinformation zu den im Befüllblister verpackten Medikamenten auf.

Bei dem erfindungsgemäßen Verfahren kann ferner ohne Einbußen an Leistungsfähigkeit der Anlage die Reihenfolge der nacheinander bestückten Befüllblister gemäß den Anforderungen der Abnehmer der befüllten Blisterpackungen festgelegt werden, wodurch Versand und Vertrieb der bestückten Blisterpackungen erheblich vereinfacht wird.

### Kurze Beschreibung der Zeichnungen

Die Erfindung wird im Folgenden anhand von Ausführungsbeispielen unter Bezugnahme auf die Zeichnungen im Detail beschrieben.
Figur 1 ist eine schematische Detailansicht von oben auf ein Ausführungsbeispiel einer erfindungsgemäßen Anlage zur personenindividuellen Bestückung von Befüllblistern.
Figur 2 zeigt ein Ausführungsbeispiel eines Befüllblisters zur Verwendung mit der erfindungsgemäßen Anlage.
Figur 3 ist eine schematische Detailansicht von oben zur Illustration des Verfahrensablaufs der Befüllung eines Befüllblisters gemäß einem Ausführungsbeispiel der Erfindung.
Figur 4 zeigt in Perspektivansicht ein Ausführungsbeispiel eines Vordosiermagazins der erfindungsgemäßen Anlage.
Figur 5 ist eine schematische Detail-Innenansicht eines Ausführungsbeispiels einer erfindungsgemäßen Medikamenten-Ausgabestation.
Figur 6 ist eine schematische Querschnittsansicht zur Illustration des Verfahrensablaufs der Befüllung eines Befüllblisters gemäß einem Ausführungsbeispiel der Erfindung.
Figur 7 ist ein Flussdiagramm zur Erläuterung der Verfahrensschritte eines Ausführungsbeispiels des erfindungsgemäßen Verfahrens zur personenindividuellen Bestückung von Befüllblistern.
Figur 8 ist eine schematische Darstellung eines Ausführungsbeispiels einer erfindungsgemäßen Anlage zur personenindividuellen Bestückung von Befüllblistern zur Erläuterung des Transports der Befüllblister in der Anlage.

### Detaillierte Beschreibung der Erfindung

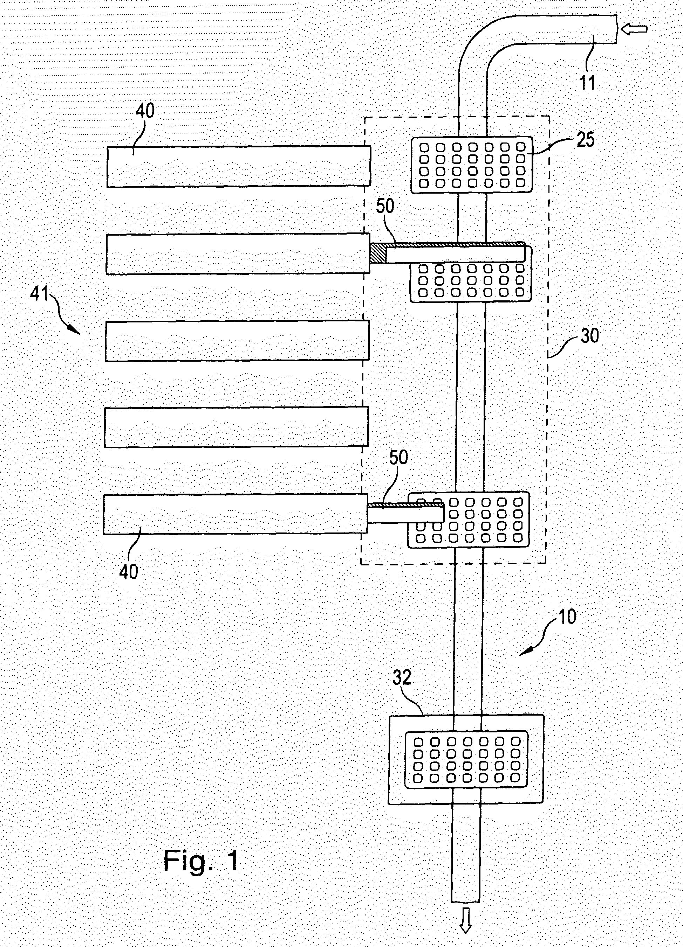

Figur 1 zeigt in schematischer Draufsicht ein Befüllmodul 41 eines Ausführungsbeispiels der erfindungsgemäßen Anlage zur personenindividuellen Bestückung von Befüllblistern 25. Ein solches Befüllmodul 41 besteht in dem dargestellten Ausführungsbeispiel aus fünf hintereinander angeordneten Medikamenten-Befüllstationen 40, die jeweils zur Ausgabe von Dosiereinheiten eines bestimmten Medikamententyps vorgesehen sind. Die Anzahl der in einem Befüllmodul 41 zusammengefassten Medikamenten-Befüllstationen 40 ist selbstverständlich nicht auf fünf beschränkt, sondern kann je nach Platzverhältnissen oder Zweckmäßigkeit gewählt werden. Ebenso ist die Gesamtzahl der Medikamenten-Befüllmodule 41 der Anlage nicht auf eine bestimmte Anzahl eingeschränkt. Die Gesamtzahl der Befüllstationen 40 bestimmt dabei im Prinzip die maximale Anzahl der mit der Anlage verblisterbaren Medikamente, wobei für besonders häufig verwendete Medikamente auch mehr als eine Befüllstation vorgesehen sein kann.

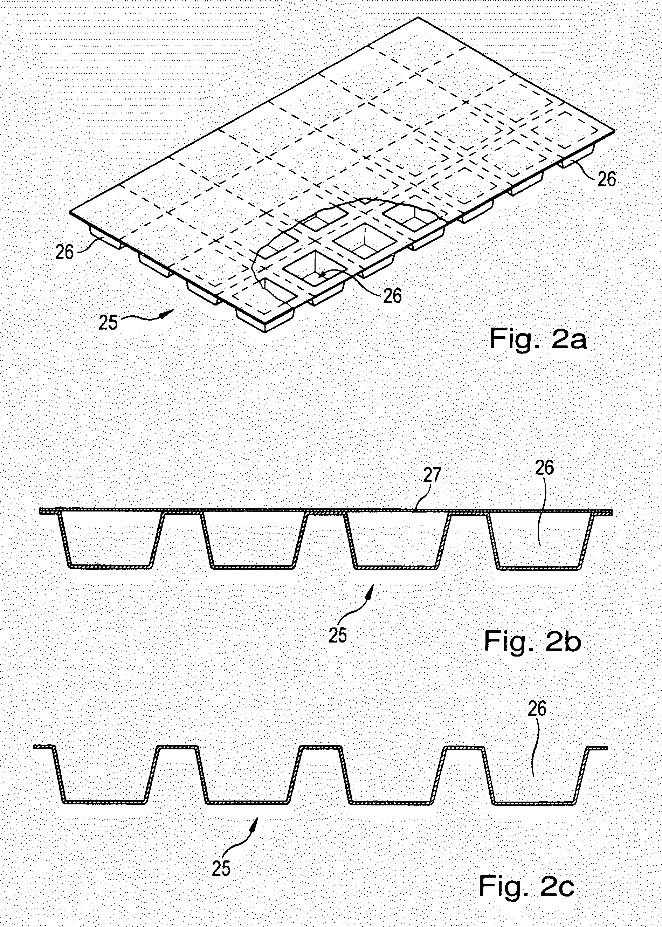

Die erfindungsgemäße Anlage dient der personenindividuellen Bestückung von Befüllblistern mit Medikamenten entsprechend vorgegebenen Verschreibungsdaten für eine bestimmte Person/Patienten. Figur 2a zeigt ein Ausführungsbeispiel eines Befüllblisters 25 zur Verwendung mit der erfindungsgemäßen Anlage in Perspektivansicht und die Figuren 2b und 2c in Querschnittsansicht mit bzw. ohne Blisterfolie 27. Wie in Figur 2 schematisch dargestellt ist, weist der Befüllblister 25 in Matrixkonfiguration angeordnete Aufnahmefächer 26 auf, welche im gezeigten Ausführungsbeispiel in vier Zeilen entsprechend einer Anzahl von Einnahmezeiten während eines Tages (morgens, mittags, abends, nachts) und sieben Spalten entsprechend den Tagen einer Woche angeordnet sind. Die Erfindung ist jedoch nicht auf diese spezielle Anordnung der Aufnahmefächer 26 des Befüllblisters eingeschränkt.

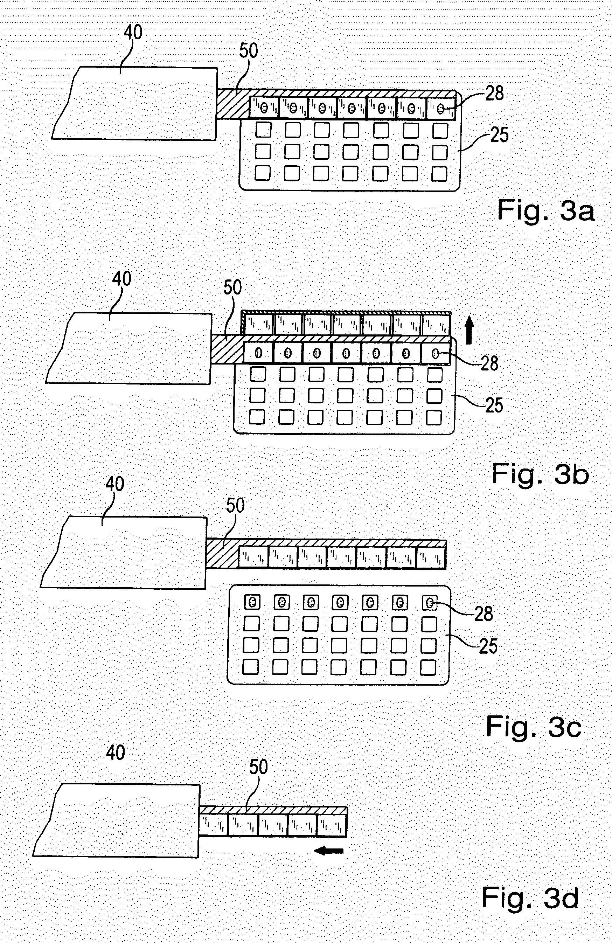

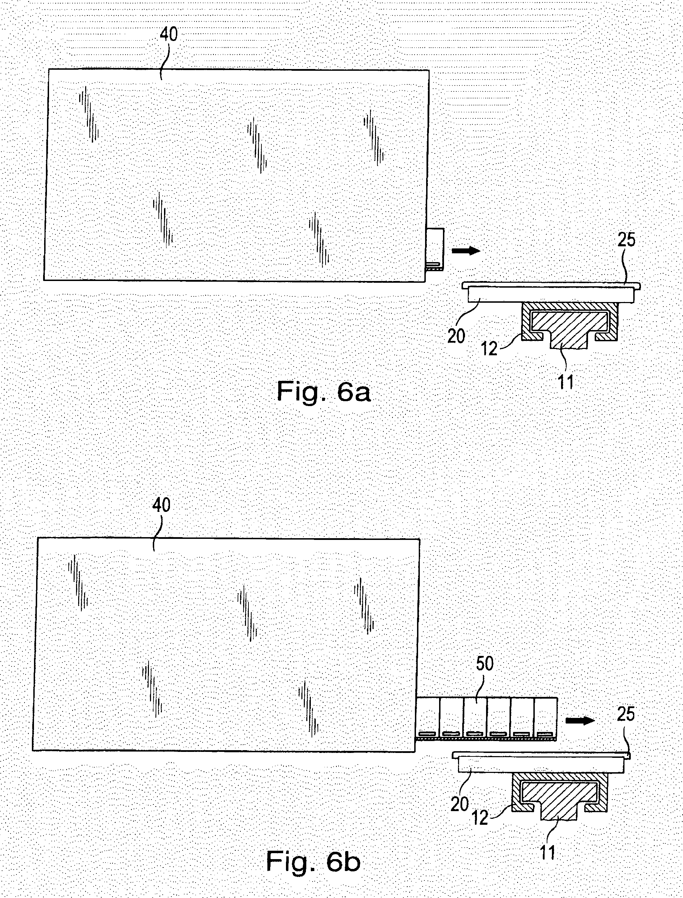

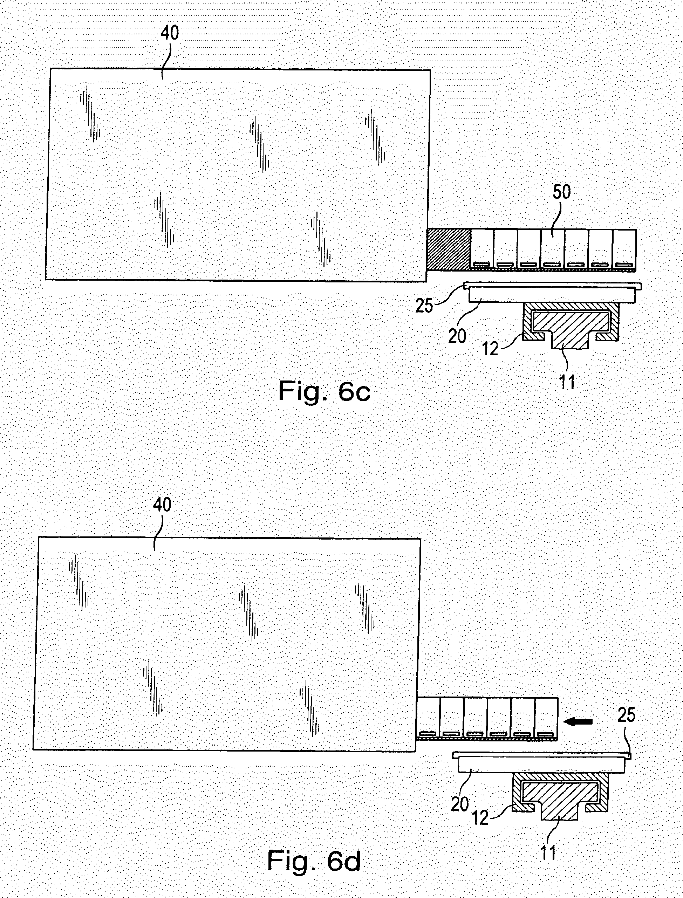

Wiederum Bezug nehmend auf Figur 1 zeigt diese eine als Magnetschiene ausgebildete Führungsschiene 11 zum Transport der Befüllblister 25, die dabei auf einem Werkstückträger 20 (schematisch dargestellt in Figur 6) aufliegen. Vorzugsweise ist der Werkstückträger 20 dazu mit (nicht dargestellten) Mulden entsprechend der Anordnung der Aufnahmefächer 26 des Befüllblisters 25 versehen. Zu Beginn des Befüllvorgangs werden die Befüllblister 25 auf den Werkstückträger 20 aufgesetzt und durchlaufen einzeln und hintereinander den gesamten Weg von der ersten Befüllstation bis zur letzten Befüllstation der Bestückungsanlage. Jeder Befüllblister wird dabei einzeln angesteuert und stoppt lediglich an denjenigen Befüllstationen 40, an denen ein Medikamenten-Befüllvorgang in den jeweiligen Befüllblister 25 entsprechend den Verschreibungsdaten des jeweiligen Patienten erforderlich ist. Typischerweise stoppt ein Befüllblister 25 so an drei bis sieben Befüllstationen, an denen das jeweilige Medikament mittels eines Vordosiermagazins 50 zeilenweise zugeführt wird, wie unter Bezugnahme auf die Figuren 3 bis 6 später im Detail erläutert werden wird. Vorzugsweise ist im Übergabebereich der Medikamente ein Reinraumbereich vorgesehen, der in Figur 1 schematisch mit 30 bezeichnet ist. Vorzugsweise wird in diesem Reinraumbereich 30 von der Decke her keimfreie und partikelarme Luft nach unten geblasen, die verhindert, dass Keime oder unerwünschte Staubpartikel in den geöffneten Befüllblister 25 gelangen.

Hinter einem Befüllmodul 41 ist ein Fototunnel 32 mit einer (nicht dargestellten) Kamera vorgesehen, die ein optisches Bild des Befüllblisters 25 nach Durchlaufen des vorangehenden Befüllmoduls 41 erstellt. Mit Hilfe des durch die Kamera im Fototunnel 32 gewonnenen Bildes des Befüllblisters kann eine Auswertungselektronik überprüfen, ob die Medikamentenbestückung entsprechend den jeweiligen Patienten-Verschreibungsdaten korrekt ausgeführt wurde.

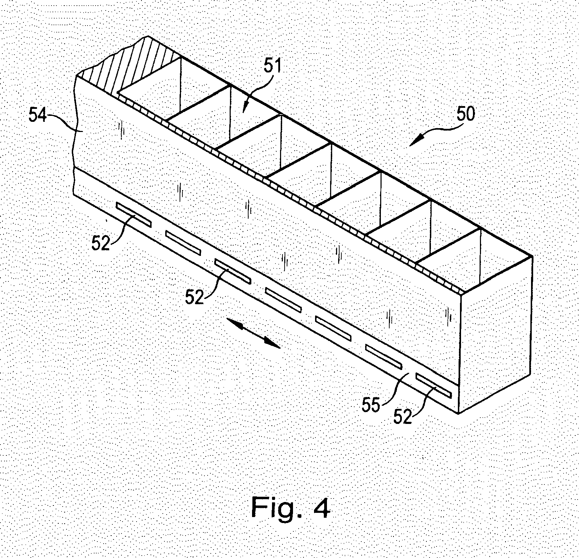

Figur 4 illustriert schematisch in Aufsicht und Figur 6 in Querschnittsansicht den Medikamenten-Befüllvorgang an einer Medikamenten-Befüllstation 40. Zunächst wird das Befüllmodul 25 mit Hilfe des Werkstückträgers 20 zu dem vorgesehenen Medikamenten-Befüllmodul 40 befördert. Das Befüllmodul 20 stoppt seine Bewegung und das Vordosiermagazin 50 führt eine Bewegung senkrecht zur Bewegungsrichtung der Befüllblister aus, wie in den Figuren 6a und 6b mittels eines Pfeils angedeutet ist. Am Ende dieser Querbewegung befindet sich jeweils ein Vordosierfach 51 über einem Wochetagsfach 26 einer Zeile des Befüllblisters 25 (Figur 3a und Figur 6c). Dann wir ein Öffnungsmechanismus der Vordosierfächer 51 des Vordosiermagazins 50 aktiviert, so dass die in den Vordosierfächern 51 befindlichen Medikamente mit geringer Fallhöhe in die entsprechenden Fächer 26 des Befüllblisters 25 fallen. Der Öffnungsmechanismus des Vordosiermagazins ist bei dem dargestellten Ausführungsbeispiel der Erfindung als verschiebbarer Boden 55 des Vordosiermagazins ausgebildet. Andere Mechanismen wie etwa Klappmechanismen oder dgl. sind im Rahmen der Erfindung jedoch ebenfalls möglich. Wird das an der Ausgabestation 40 "verabreichte" Medikament 28 noch in einer anderen Tageszeile des Befüllblisters 25 benötigt (beispielsweise für eine Medikamenteneinnahme morgens und abends) bleibt der Befüllblister in Position (nicht ganz, er wird schon um eine entsprechende Anzahl von Zeilen weitertransportiert), das Vordosiermagazin 50 fährt zurück (Figur 3d und Figur 6d), wird mit einer erneuten Portionierung der Medikamente versehen, die dann auf die gleiche Art und Weise in die weitere Tageszeitzeite des Befüllblisters 25 eingefüllt werden. Wenn der Bestückungsvorgang mit diesem bestimmten Medikament abgeschlossen ist, fährt der Befüllblister 25 zur nächsten vorgesehen Medikamenten-Befüllstation 40.

Ein Ausführungsbeispiel eines Vordosiermagazins 50 ist in schematischer Perspektivansicht in Figur 4 gezeigt. Es sind insgesamt sieben Vordosierfächer 71 entsprechend den sieben Wochentagsspalten des Befüllblisters 25 vorgesehen, wobei erfindungsgemäß auch eine andere Anzahl an Fächern 51 möglich ist. Vorzugsweise sind die Vordosierfächer 51 in Form einer transparenten, zur Reinigungszwecken austauschbaren Innenverkleidung aus Kunststoff ausgebildet, die an einem vorzugsweise aus Metall ausgeführten stabilen Rahmenteil 54 befestigbar ist. Im unteren Bereich der Seitenwände der Vordosierfächer 51 sind beidseitig Schlitze 52 vorgesehen zur optischen Überprüfung, ob sich in dem jeweiligen Vordosierfach ein Medikament befindet oder nicht.

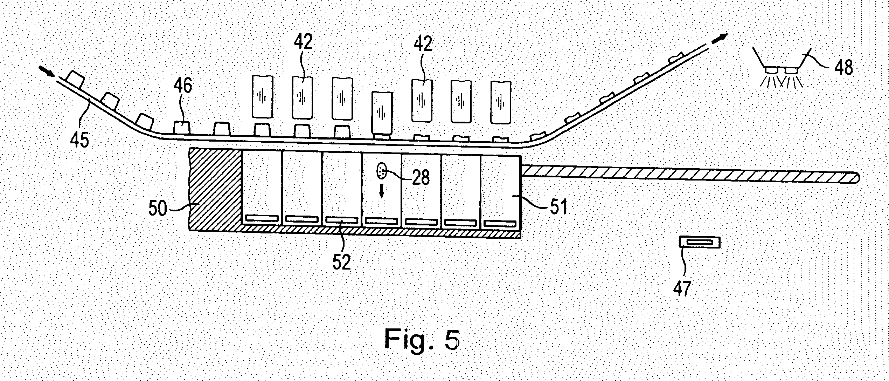

Der Mechanismus zur Zuführung der Medikamente 28 in das Vordosiermagazin 50 einer Medikamenten-Befüllstation 40 ist schematisch in Figur 5 dargestellt. In dem gezeigten Ausführungsbeispiel wird das Medikament mittels eines Endlos-Streifenblisters 45 zugeführt, der äquidistant angeordnete Blisterhöfe 46 zur Aufnahme der Medikamentendosierungen aufweist, wobei der Abstand der einzelnen Blisterhöfe 46 des Streifenblisters 45 voneinander dem Abstand der Vordosierfächer 51 des Vordosiermagazins 50 entsprechen kann. Der in den jeweiligen Medikamenten-Befüllstationen 40 befindliche Streifenblister 45 wird vorzugsweise mit Hilfe eines (nicht dargestellten) Antriebsmechanismus von einer an sich bekannten Rolle abgerollt. Ist ein Streifenblister 45 aufgebraucht, so kann ohne Schwierigkeiten eine neue, gefüllte Streifenblisterrolle in der Medikamenten-Befüllstation 40 eingehängt werden und/oder der neue Streifenblister direkt an den vorhandenen Streifenblister angeklebt oder angeheftet werden, so dass eine unterbrechungsfreie Versorgung mit Medikamenten gewährleistet ist. Für sehr selten verschriebene Medikamente, für die sich eine Streifenverblisterung nicht lohnt, kann wenigstens eine Medikamenten-Befüllstation 40 zur manuellen Befüllung des Vordosiermagazins 50 vorgesehen sein. Dadurch wird es möglich, die Flexibilität der Anlage zu erhöhen und auch sehr selten verschriebene Medikamente zu wirtschaftlichen Bedingungen in die Verblisterung mit aufzunehmen.

Wie in Figur 5 dargestellt, weist jede Medikamenten-Befüllstation 40 Auswurfstößel 42 auf zum Ausstoßen der Medikamente 28 aus dem Streifenblister 45 in das Vordosiermagazin 50. Vorzugsweise sind die Auswurfstößel 42 in einer Anzahl entsprechend der Anzahl der Vordosierfächer 51 des Vordosiermagazins 50 zum zeitsparenden parallelen Ausstoßen der Medikamenteneinheiten vorgesehen; dies ist jedoch nicht unbedingt erforderlich, sondern ein sequenzielles Ausstoßen der Medikamente ist ebenso möglich.

Vorzugsweise weist eine Medikamenten-Befüllstation 40 wenigstens zwei Sensorvorrichtungen 47, 48 zur Überprüfung der korrekten Medikamentenübergabe Streifenblister-Vordosiermagazin-Befüllblister auf. Eine erste Durchlichtkamera oder Lichtschranke 47 erfasst durch die an den Vordosierfächern 51 vorgesehenen Schlitze 52, ob sich während der Ausfahrbewegung des Vordosiermagazins 50 ein Medikament in dem entsprechenden Vordosierfach 51 befindet und ob es sich bei der späteren Einfahrbewegung nicht mehr darin befindet, wodurch die korrekte Abgabe der Medikamenteneinheiten in die entsprechenden Fächer des Befüllblisters validiert werden kann. Zusätzlich ist vorzugsweise eine Auflichtkamera 48 vorgesehen, die den Befüllzustand der Vordosierfächer 51 vor der Medikamentenübergabe an den Befüllblister und danach ebenfalls optisch validiert, entweder mittels Lichtreflexion oder als Durchlichtdetektor. In letzterem Fall muss der Boden des Vordosiermagazins 50 lichtdurchlässig ausgebildet sein.

Gegenüber dem aus der WO 2005 102 841 A1 bekannten Stand der Technik sorgt die Verwendung des Vordosiermagazins 50 bei der erfindungsgemäßen Bestückungsanlage dafür, dass der Ausstoßvorgang aus dem Streifenblister 45 und der Bestückungsvorgang des Befüllblisters 25 voneinander entkoppelt werden. Dadurch kann der für die Leistungsfähigkeit der Gesamtanlage zeitkritische Befüllvorgang des Befüllblisters 25 in Bezug auf Schnelligkeit optimiert werden, während für den mechanisch schwierig auszuführenden Ausstoßvorgang aus dem Streifenblister 45 mehr Zeit zur Verfügung steht, ohne die Leistungsfähigkeit der Gesamtanlage zu beeinträchtigen.

Zur Förderung der Werkstückträger 12 ist erfindungsgemäß vorzugsweise ein Magnetschienensystem 10 mit Linearmotorantrieb vorgesehen, weiches es erlaubt, jeden Werkstückträger 20 und damit den darauf befindlichen Befüllblister 25 einzeln zu steuern. Das Magnetschienensystem 10 weist eine in sich geschlossene Führungsschiene 11 auf, die in Querschnittsansicht in Figur 6 gezeigt ist. Jeder Werkstückträger 20 weist ein Führungselement 12 auf, das die Führungsschiene 11 umgreift und auf an sich bekannte Art und Weise nach dem Prinzip eines Magnetschwebezuges berührungslos oder berührend (gleitend, rollend) in der Führungsschiene 11 geführt wird. Ein ebenfalls an sich bekannter elektrischer Linearmotor sorgt für den einzeln ansteuerbaren Antrieb der Werkstückträger 20 und damit der Befüllblister 25.

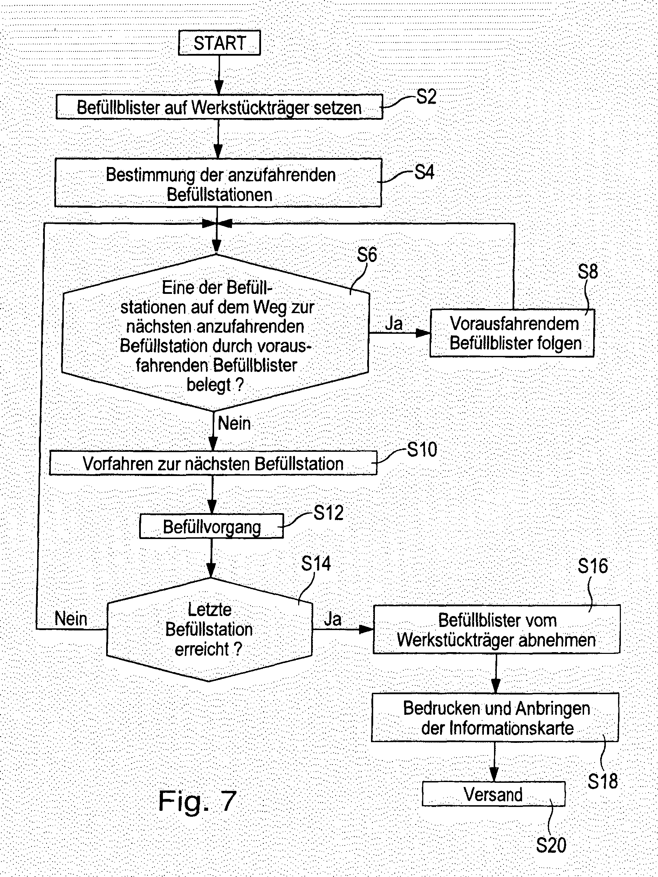

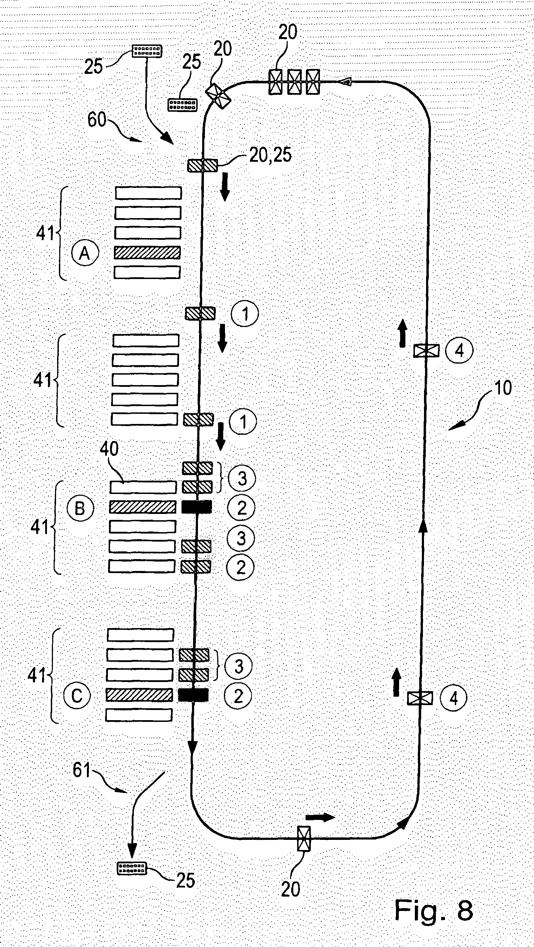

Ein Ausführungsbeispiel des erfindungsgemäßen Verfahrens zur personenindividuellen Bestückung von Befüllblistern wird im Folgenden unter Bezugnahme auf die Figuren 7 und 8 erläutert.

Zu Beginn der Bestückungsstrecke vor dem ersten Befüllmodul 41 ist eine Befüllblister-Auflagestation 60 vorgesehen, die in Figur 8 schematisch angedeutet ist. Am Ende der Bestückungsstrecke ist entsprechend eine Befüllblister-Entnahmestation 61 angeordnet.

In einem ersten Verfahrensschritt S2 (Figur 7) wird somit in der Befüllblister-Auflagestation 60 ein leerer Befüllblister 25 auf den Werkstückträger 20 gesetzt. Dann (oder gleichzeitig) werden in Verfahrensschritt S4 die anzufahrenden Befüllstationen bestimmt, in dem in Figur 8 gezeigten Ausführungsbeispiel sind dies die Stationen A, B und C. Für den betrachteten Befüllblister 25 wird nun in Verfahrensschritt S6 überprüft, ob der Transportweg zur nächsten anzufahrenden Befüllstation durch einen an einer dazwischenliegenden Befüllstation in einen Befüllvorgang verwickelten vorausfahrenden Befüllblister belegt ist oder nicht. Ist dies der Fall, so folgt bzw. wartet der betrachtete Befüllblister 25 dem vorausgehenden Befüllblister gemäß Verfahrensschritt S8. Ist der Weg zur nächsten anzufahrenden Befüllstation 40 hingegen frei, fährt der Befüllblister 25 gemäß Schritt S10 mit maximaler Transportgeschwindigkeit zur dieser nächsten Medikamenten-Befüllstation 40, wo in Verfahrensschritt S12 der Befüllvorgang ausgeführt wird. Die Verfahrensschritte S6 bis S12 wiederholen sich, bis in Schritt S14 festgestellt wird, dass die letzte vorgesehene Befüllstation erreicht ist. Dann wird in Verfahrensschritt S16 der Befüllblister an der Befüllblister-Entnahmestation 61 entnommen, der Befüllblister 25 mit der Blisterfolie 27 versiegelt und eine (nicht dargestellte) Informationskarte mit Patienten-Identifizierungsinformation sowie Verschreibungsinformation für den Patienten gedruckt und diese Informationskarte an den Blister angebracht. Schließlich ist der bestückte und mit Informationskarte versehene Befüllblister 25 fertig für den Versand (Schritt S20).

Figur 8 zeigt schematisch die verschiedenen Bewegungszustände der Befüllblister. Bewegungszustand (1), illustriert durch ein schraffiertes Rechteck mit Pfeil, bezeichnet eine Fortbewegung mit Maximalgeschwindigkeit bis zum nächsten anzusteuernden Medikamenten-Befüllstation oder bis zu einer "Warteschlange" vorausfahrender Befüllblister, Bewegungszustand (2), illustriert durch ein ausgefülltes Rechteck, bezeichnet einen Befüllvorgang an einer Medikamenten-Befüllstation 40, Bewegungszustand (3), illustriert durch ein schraffiertes Rechteck ohne Pfeil, bezeichnet eine Fortbewegung des Befüllblisters in einer "Warteschlange" hinter einem vorausfahrenden Befüllblister, und Bewegungszustand (4), illustriert durch mit Kreuz versehenes Rechteck, bezeichnet die Rückfahrbewegung eines leeren Werkstückträgers 20 zur Befüllblister-Auflagestation 60. Da die Anzahl der von jedem einzelnen Blister anzusteuernden Befüllstationen im Verhältnis zur Gesamtzahl der Befüllstationen relativ gering ist, sind die mit Maximalgeschwindigkeit zurücklegbaren Strecken (Bewegungszustand (1)) relativ lang, so dass die Leistungsfähigkeit der Gesamtanlage im Vergleich zu einer Anlage mit taktweisem Betrieb wie in der WO 2005 102 841 A1 offenbart, erheblich verbessert werden kann. Dies gilt auch dann, wenn die Reihenfolge der einzelnen Befüllblister 25 nicht bezüglich der Verblisterung sondern in Bezug auf die Versandreihenfolge an die Endabnehmer (Apotheken) optimiert ist.

Die Erfindung ermöglicht durch die individuelle Steuerung der einzelnen Befüllblister nur an die entsprechend den Verschreibungsdaten jeweils erforderlichen Medikamenten-Befüllstationen einen im Vergleich zum herkömmlichen Taktbetrieb erheblich schnelleren Durchlauf der einzelnen Befüllblister durch die Anlage und damit eine höhere Leistungsfähigkeit. Falls eine Befüllstation 40 aufgrund von Wartungsarbeiten oder des Wechsels des Streifenblisters kurzzeitig ausfällt, erfordert dies nicht die Stillegung der Gesamtanlage. Auch der Austausch einer defekten Befüllstation durch eine neue Befüllstation ist problemlos möglich, ohne die Gesamtanlage herunterzufahren.

## Patentansprüche

1. Anlage zur personenindividuellen Bestückung von Befüllblistern (25) mit Medikamenten entsprechend vorgegebenen Verschreibungsdaten, wobei die Befüllblister (25) in einer Matrixkonfiguration in Zeilen entsprechend einer Anzahl von Einnahmezeiten während eines Tages und Spalten entsprechend einer Anzahl von Tagen, beispielsweise Wochentagen, angeordnete Aufnahmefächer für jeweilige Medikamenten-Verabreichungseinheiten aufweisen, umfassend:
eine Vielzahl hintereinander angeordneter Medikamenten-Befüllstationen (40) zur Bestückung der Befüllblister (25) mit jeweils einem bestimmten Medikament,
eine Transportvorrichtung (10) ausgebildet zum Transport der Befüllblister (25) einzeln hintereinander in einer Bewegungsrichtung entlang der Medikamenten-Befüllstationen (40), wobei jeder Befüllblister (25) individuell gesteuert ist, derart dass dieser nur die entsprechend den Verschreibungsdaten jeweils erforderlichen Medikamenten-Befüllstationen (40) für einen Befüllvorgang anfährt und die anderen Medikamenten-Befüllstationen (40) passiert und direkt bis zur nächsten erforderlichen Medikamenten-Befüllstationen (40) oder zu einer Position hinter einem an einer Medikamenten-Befüllstationen (40) befindlichen vorausgehenden Befüllblister (25) weiterbefördert wird, **dadurch gekennzeichnet, dass** die Befüllblister (25) einzeln und hintereinander den gesamten Weg von der ersten bis zur letzten der Medikamenten-Befüllstationen (40) durchlaufen und die Geschwindigkeit der beförderten Befüllblister (25) einzeln ansteuerbar ist.

2. Anlage nach Anspruch 1, wobei die Transportvorrichtung (10) als Magnetbahn mit Linearmotor zum Antrieb der Befüllblister (25) ausgebildet ist.

3. Anlage nach Anspruch 1 oder 2, wobei die Transportvorrichtung (10) zum Transport von in einer Magnetschiene (11) geführten Werkstückträgern (20) zur Aufnahme der Befüllblister (25) ausgebildet ist.

4. Anlage nach Anspruch 3, wobei die Transportvorrichtung (10) als geschlossene Bahn ausgebildet ist, auf der die Werkstückträger (20) in einem geschlossenen Kreislauf gefördert werden, wobei die Befüllblister (25) vor der Bestückung auf die Werkstückträger (20) aufgesetzt und nach dem Bestückungsvorgang wieder entnommen werden.

5. Anlage nach einem der vorhergehenden Ansprüche, wobei in einem Arbeitsschritt jeweils eine Spalte eines Befüllblisters (25) entsprechend einer Anzahl von Tagen gleichzeitig befüllt wird.

6. Anlage nach einem der vorhergehenden Ansprüche, wobei jede Befüllstation (40) ein bewegbares Vordosiermagazin (50) zur Befüllung der Befüllblister (25) aufweist.

7. Anlage nach einem der vorhergehenden Ansprüche, ferner umfassend eine über der Transportvorrichtung (10) angeordnete Kamera zur optischen Überprüfung der korrekten Bestückung der Befüllblister (25) entsprechend den Verschreibungsdaten.

8. Anlage nach Anspruch 7, wobei jeweils mehrere Befüllstationen (40) zu Befüllmodulen (41) zusammengefasst sind und nach jedem Befüllmodul (41) eine Kamera zur optischen Überprüfung der korrekten Bestückung der Befüllblister (25) angeordnet ist.

9. Anlage nach einem der vorhergehenden Ansprüche, aufweisend einen Reinraumbereich (30) zur Schaffung von Reinraumbedingungen bei der Bestückung der Befüllblister (25).

10. Verfahren zur personenindividuellen Bestückung von Befüllblistern (25) mit Medikamenten entsprechend vorgegebenen Verschreibungsdaten, wobei die Befüllblister (25) in einer Matrixkonfiguration in Zeilen entsprechend einer Anzahl von Einnahmezeiten während eines Tages und Spalten entsprechend einer Anzahl von Tagen, beispielsweise Wochentagen, angeordnete Aufnahmefächer für jeweilige Medikamenten-Verabreichungseinheiten aufweisen, welches Verfahren umfasst:
Förderung der Befüllblister (25) einzeln hintereinander in einer Bewegungsrichtung entlang von hintereinander angeordneten Medikamenten-Befüllstationen (40) zur Bestückung der Befüllblister (25) mit jeweils einem bestimmten Medikament, wobei jeder Befüllblister (25) individuell gesteuert wird, derart dass dieser nur die entsprechend den Verschreibungsdaten jeweils erforderlichen Medikamenten-Befüllstationen (40) für einen Befüllvorgang anfährt und die anderen Medikamenten-Befüllstationen (40) passiert und direkt bis zur nächsten erforderlichen Medikamenten-Befüllstationen (40) oder zu einer Position hinter einem an einer Medikamenten-Befüllstationen (40) befindlichen vorausgehenden Befüllblister (25) weiterbefördert wird, **dadurch gekennzeichnet, dass** die Befüllblister (25) einzeln und hintereinander den gesamten Weg von der ersten bis zur letzten der Medikamenten-Befüllstationen (40) durchlaufen und die Geschwindigkeit der beförderten Befüllblister (25) einzeln ansteuerbar ist.

11. Verfahren nach Anspruch 10, wobei die Befüllblister (25) mittels einer Magnetbahn (10) mit Linearmotor angetrieben werden.

12. Verfahren nach Anspruch 10 oder 11, wobei Werkstückträger (20) zur Aufnahme der Befüllblister (25) vorgesehen sind, welche Werkstückträger (20) in einem geschlossenen Kreislauf gefördert werden, wobei die Befüllblister (25) vor der Bestückung auf die Werkstückträger (20) aufgesetzt und nach dem Bestückungsvorgang wieder entnommen werden.

13. Verfahren nach Anspruch 10, 11 oder 12, wobei in einem Arbeitsschritt jeweils eine Spalte eines Befüllblisters (25) entsprechend einer Anzahl von Tagen gleichzeitig befüllt wird.

14. Verfahren nach einem der Ansprüche 10 bis 13, wobei die Befüllblister (25) mittels eines bewegbaren Vordosiermagazins (50) befüllt werden.

15. Verfahren nach einem der Ansprüche 10 bis 14, ferner umfassend den Verfahrensschritt der optischen Überprüfung der korrekten Bestückung der Befüllblister (25) entsprechend den Verschreibungsdaten.

16. Verfahren nach einem der Ansprüche 10 bis 15, wobei die Bestückung der Befüllblister (25) unter Reinraumbedingungen erfolgt.

17. Verfahren nach einem der Ansprüche 10 bis 16, umfassend den Verfahrensschritt des Erstellens und Anfügens eines Dokumentationsträgers an den Befüllblister (25) umfassend personenbezogene Information und/oder Anwendungsinformation zu den im Befüllblister (25) verpackten Medikamenten.

18. Verfahren nach einem der Ansprüche 10 bis 17, wobei die Reihenfolge der nacheinander bestückten Befüllblister (25) gemäß den Anforderungen der Abnehmer der befüllten Blisterpackungen festgelegt wird.

## Claims

1. System for personalised filling of filling blisters (25) with medicaments in accordance with specified prescription data, the filling blisters (25) having receiving compartments for respective medicament administration units, arranged in a matrix configuration in rows corresponding to a number of taking times during a day and columns corresponding to a number of days, for example weekdays, comprising:
a multiplicity of medicament filling stations (40) arranged one behind the other, for filling the filling blisters (25) with a respective particular medicament, a transport device (10) configured for transporting the filling blisters (25) individually one behind the other in a movement direction along the medicament filling stations (40), each filling blister (25) being individually controlled in such a way that it stops only at the medicament filling stations (40) respectively required in accordance with the prescription data for a filling operation and passes the other medicament filling stations (40) and is conveyed on directly to the next required medicament filling stations (40) or to a position behind a filling blister (25) running ahead and situated at a medicament filling stations (40), **characterised in that** the filling blisters (25) travel, individually and one behind the other, over the entire path from the first to the last of the medicament filling stations (40) and the speed of the conveyed filling blisters (25) is individually controllable.

2. System according to Claim 1, the transport device (10) being configured as a magnetic track with a linear motor for driving the filling blisters (25).

3. System according to Claim 1 or 2, the transport device (10) being configured to transport workpiece carriers (20), guided in a magnetic rail (11), for receiving the filling blisters (25).

4. System according to Claim 3, the the transport device (10) being configured as a closed track, on which the workpiece carriers (20) are conveyed in a closed circuit, the filling blisters (25) being placed on the workpiece carriers (20) before the filling and removed again after the filling operation.

5. System according to one of the preceding claims, in a processing step respectively one column of a filling blister (25) being simultaneously filled corresponding to a number of days.

6. System according to one of the preceding claims, each filling station (40) having a movable pre-dosing magazine (50) for filling the filling blisters (25).

7. System according to one of the preceding claims, further comprising a camera, arranged above the transport device (10), for optical checking of the correct filling of the filling blisters (25) in accordance with the prescription data.

8. System according to Claim 7, in each case a plurality of filling stations (40) being combined to form filling modules (41) and after each filling module (41) there being arranged a camera for optical checking of the correct filling of the filling blisters (25).

9. System according to one of the preceding claims, having a clean-room area (30) for creating clean-room conditions during the filling of the filling blisters (25).

10. Method for personalised filling of filling blisters (25) with medicaments in accordance with specified prescription data, the filling blisters (25) having receiving compartments for respective medicament administration units, arranged in a matrix configuration in rows corresponding to a number of taking times during a day and columns corresponding to a number of days, for example weekdays, which method comprises:
conveying the filling blisters (25) individually one behind the other in a movement direction along medicament filling stations (40) arranged one behind the other for filling the filling blisters (25) with a respective particular medicament, each filling blister (25) being individually controlled in such a way that it stops only at the medicament filling stations (40) respectively required in accordance with the prescription data for a filling operation and passes the other medicament filling stations (40) and is conveyed on directly to the next required medicament filling stations (40) or to a position behind a filling blister (25) running ahead and situated at a medicament filling stations (40), **characterised in that** the filling blisters (25) travel, individually and one behind the other, over the entire path from the first to the last of the medicament filling stations (40) and the speed of the conveyed filling blisters (25) is individually controllable.

11. Method according to Claim 10, the filling blisters (25) being driven by means of a magnetic track (10) with a linear motor.

12. Method according to Claim 10 or 11, workpiece carriers (20) for receiving the filling blisters (25) being provided, which workpiece carriers (20) are conveyed in a closed circuit, the filling blisters (25) being placed on the workpiece carriers (20) before the filling and removed again after the filling operation.

13. Method according to Claim 10, 11 or 12, in a processing step respectively one column of a filling blister (25) being simultaneously filled corresponding to a number of days.

14. Method according to one of Claims 10 to 13, the filling blisters (25) being filled by means of a movable pre-dosing magazine (50).

15. Method according to one of Claims 10 to 14, further comprising the method step of optical checking of the correct filling of the filling blisters (25) in accordance with the prescription data.

16. Method according to one of Claims 10 to 15, the filling of the filling blisters (25) being performed under clean-room conditions.

17. Method according to one of Claims 10 to 16, comprising the method step of creating and attaching a documentation carrier to the filling blister (25) comprising personal information and/or use information on the medicaments packaged in the filling blister (25).

18. Method according to one of Claims 10 to 17, the order of the successively filled filling blisters (25) being set in accordance with the requirements of the purchasers of the filled blister packs.

## Revendications

1. Installation destinée au garnissage pour une personne de blisters de remplissage (25) avec des médicaments en fonction de données de prescription prédéfinies, les blisters de remplissage (25) présentant des cases de réception destinées à des unités d'administration de médicaments respectives et disposées, dans une configuration matricielle, en lignes en fonction d'un nombre d'horaires de prise pendant une journée et en colonnes en fonction d'un nombre de jours, par exemple de jours de la semaine, comprenant :
une multiplicité de postes de remplissage en médicaments (40) disposés les uns derrière les autres pour le garnissage des blisters de remplissage (25) avec respectivement un médicament déterminé,
un dispositif de transport (10) constitué pour le transport des blisters de remplissage (25) individuellement les uns derrière les autres dans une direction de mouvement le long des postes de remplissage en médicaments (40), chaque blister de remplissage (25) étant commandé individuellement de telle sorte qu'il ne se dirige pour un processus de remplissage que vers les postes de remplissage en médicaments (40) respectivement nécessaires en fonction des données de prescription et qu'il dépasse les autres postes de remplissage en médicaments (40) et est transporté plus loin directement jusqu'au poste de remplissage en médicaments (40) nécessaire suivant ou vers une position derrière un blister de remplissage (25) précédent situé au niveau d'un poste de remplissage en médicaments (40), **caractérisée en ce que** les blisters de remplissage (25) parcourent individuellement les uns derrière les autres la totalité du chemin depuis le premier jusqu'au dernier des postes de remplissage en médicaments (40), et **en ce que** la vitesse des blisters de remplissage (25) acheminés peut être pilotée individuellement.

2. Installation selon la revendication 1, le dispositif de transport (10) étant constitué en tant que piste magnétique avec moteur linéaire pour l'entraînement des blisters de remplissage (25).

3. Installation selon la revendication 1 ou 2, le dispositif de transport (10) étant constitué pour le transport de porte-pièces (20) guidés dans un rail magnétique (11) pour la réception des blisters de remplissage (25).

4. Installation selon la revendication 3, le dispositif de transport (10) étant constitué en tant que piste fermée sur laquelle les porte-pièces (20) sont acheminés dans un circuit fermé, les blisters de remplissage (25) étant placés sur les porte-pièces (20) avant le garnissage et puis prélevés de nouveau après le processus de garnissage.

5. Installation selon une des revendications précédentes, une colonne d'un blister de remplissage (25) étant respectivement, en une étape de travail, remplie simultanément en fonction d'un nombre de jours.

6. Installation selon une des revendications précédentes, chaque poste de remplissage (40) présentant un magasin de prédosage (50) mobile pour le remplissage des blisters de remplissage (25).

7. Installation selon une des revendications précédentes, comprenant en outre une caméra disposée au-dessus du dispositif de transport (10) pour le contrôle optique du garnissage correct des blisters de remplissage (25) en fonction des données de prescription.

8. Installation selon la revendication 7, plusieurs postes de remplissage (40) étant respectivement réunis en modules de remplissage (41), et une caméra étant disposée derrière chaque module de remplissage (41) pour le contrôle optique du garnissage correct des blisters de remplissage (25).

9. Installation selon une des revendications précédentes, présentant une zone de salle blanche (30) pour la création de conditions de salle blanche lors du garnissage des blisters de remplissage (25).

10. Procédé destiné au garnissage pour une personne de blisters de remplissage (25) avec des médicaments en fonction de données de prescription prédéfinies, les blisters de remplissage (25) présentant des cases de réception destinées à des unités d'administration de médicaments respectives et disposées, dans une configuration matricielle, en lignes en fonction d'un nombre d'horaires de prise pendant une journée et en colonnes en fonction d'un nombre de jours, par exemple de jours de la semaine, lequel procédé comprend :
l'acheminement des blisters de remplissage (25) individuellement les uns derrière les autres dans une direction de mouvement le long de postes de remplissage en médicaments (40) disposés les uns derrière les autres pour le garnissage des blisters de remplissage (25) avec respectivement un médicament déterminé, chaque blister de remplissage (25) étant commandé individuellement de telle sorte qu'il ne se dirige pour un processus de remplissage que vers les postes de remplissage en médicaments (40) respectivement nécessaires en fonction des données de prescription et qu'il dépasse les autres postes de remplissage en médicaments (40) et est transporté plus loin directement jusqu'au poste de remplissage en médicaments (40) nécessaire suivant ou vers une position derrière un blister de remplissage (25) précédent situé à un poste de remplissage en médicaments (40), **caractérisé en ce que** les blisters de remplissage (25) parcourent individuellement les uns derrière les autres la totalité du chemin depuis le premier jusqu'au dernier des postes de remplissage en médicaments (40) et **en ce que** la vitesse des blisters de remplissage (25) acheminés peut être pilotée individuellement.

11. Procédé selon la revendication 10, les blisters de remplissage (25) étant entraînés au moyen d'une piste magnétique (10) avec moteur linéaire.

12. Procédé selon la revendication 10 ou 11, des porte-pièces (20) étant prévus pour la réception des blisters de remplissage (25), lesquels porte-pièces (20) sont acheminés dans un circuit fermé, les blisters de remplissage (25) étant, avant le garnissage, placés sur les porte-pièces (20) et puis prélevés de nouveau après le processus de remplissage.

13. Procédé selon la revendication 10, 11 ou 12, une colonne d'un blister de remplissage (25) étant respectivement, en une étape de travail, garnie simultanément en fonction d'un nombre de jours.

14. Procédé selon une des revendications 10 à 13, les blisters de remplissage (25) étant remplis au moyen d'un magasin de prédosage (50) mobile.

15. Procédé selon une des revendications 10 à 14, comprenant également l'étape de procédé de contrôle optique du garnissage correct des blisters de remplissage (25) en fonction des données de prescription.

16. Procédé selon une des revendications 10 à 15, le garnissage des blisters de remplissage (25) s'effectuant dans des conditions de salle blanche.

17. Procédé selon une des revendications 10 à 16, comprenant l'étape de procédé d'établissement et d'ajout sur le blister de remplissage (25) d'un support de documentation comprenant une information de caractère personnel et/ou une information d'utilisation concernant les médicaments emballés dans le blister de remplissage (25).

18. Procédé selon une des revendications 10 à 17, l'ordre de succession des blisters de remplissage (25) garnis les uns après les autres étant fixé conformément aux exigences des acheteurs des blisters remplis.
